Europäisches Patentamt

European Patent Office

Office européen des brevets

(11) Publication number: **0 398 076**
**A1**

# EUROPEAN PATENT APPLICATION

(21) Application number: 90108343.6

(51) Int. Cl.5: **A61B 5/12**

(22) Date of filing: **03.05.90**

(30) Priority: **15.05.89 CA 599641**

(43) Date of publication of application:
**22.11.90 Bulletin 90/47**

(84) Designated Contracting States:
**BE DE DK ES FR GB IT NL SE**

(71) Applicant: **Madsen, Poul Bent**
**2569 Misener Crescent**
**Mississauga, Ontario L5K 1M9(CA)**

(72) Inventor: **Madsen, Poul Bent**
**2569 Misener Crescent**
**Mississauga, Ontario L5K 1M9(CA)**

(74) Representative: **Roerboel, Leif et al**
**c/o Dansk Patent Kontor A/S H.C.**
**Oerstedsvej 70 Postbox 237**
**DK-1879 Frederiksberg C(DK)**

(54) Acoustic impedance measurement probe.

(57) A probe for use in acoustic impedance measurement is disclosed. The probe has a probe element (4) with a distal end for insertion in a patient's ear canal, an actuator (5) coaxial with and outside the probe element, and a flexible cuff (1) outside and extending between the distal ends (3, 7) of the probe element and the actuator. The actuator is moveable axially with respect to the probe element between a first position where the distal ends are axially separated such that the cuff is in an unexpanded minimum diameter state and a second position where the distal ends are closer together such that the cuff is forced to an expanded larger diameter state, the cuff being sufficiently flexible to effect a seal between it and the patient's ear canal.

Fig. 1

## ACOUSTIC IMPEDANCE MEASUREMENT PROBE

### TECHNICAL FIELD

This invention relates to a probe for use in acoustic impedance measurement, a method of diagnosing abnormalities in the middle ear, cochlea and the nervous system.

To measure these functions, it is known in the prior art to insert a probe into the external ear canal. The probe is connected to a microphone and miniature loudspeaker as well as to an air pump. The pump can change the air pressure in the external ear canal, normally between + 400 and -600 daPa. The probe has to be fitted into the ear canal in an airtight fashion in order to set up different pressures within the ear canal.

### BACKGROUND ART

Since electroacoustic impedance was introduced in the early 1960's, one of the biggest problems has been to obtain an airtight seal in the ear canal. Different methods have been used. First, an inflatable balloon was fitted to the probe and by inflating the balloon it was possible to obtain an airtight seal. The problem was that the balloons would in some case puncture. The "bang" from the puncture could be so loud (because the balloon was very close to the tympanic membrane) that the patient in many cases had his or her hearing damaged. The next step was to fill the balloon with a liquid, but this method never became popular because if the balloon punctured, the liquid filled the ear canal and ran out on the patient's clothes. It could also be dangerous for the patient if he or she had a perforation of the tympanic membrane, as the fluid could then enter the middle ear. After that, a mushroom type of ear tip was introduced. There was a range of sizes from 6 to 14 mm in diameter, and one had to choose the size that would fit the ear canal. This is troublesome, and if the ear canal is not circular it can be very difficult to obtain a seal. There are also surface ear tips. These tips do not go into the ear canal but attempt to make a seal to the outer ear. These can only be used for rapid screening tests as they have to be held in place by the operator of the instrument. When diagnoses are made it is a requirement that the probe can be placed in the ear canal and remain there without anybody touching it.

### DISCLOSURE OF THE INVENTION

It its an object of the invention to provide an improved probe for use in acoustic impedance measurement, to address the problems of the prior art devices.

Thus in accordance with the present invention there is provided a probe for use in acoustic impedance measurement, having a probe element with a distal end for insertion in a patient's ear canal, an actuator coaxial with and outside the probe element, the actuator having a distal end not extending as far as the distal end of the probe element, and a flexible cuff outside and extending between the distal ends of the probe element and the actuator. The actuator is moveable axially with respect to the probe element between a first position where the distal ends are axially separated such that the cuff is in an unexpanded minimum diameter state and a second position where the distal ends are closer together such that the cuff is forced to an expanded larger diameter state, the cuff being sufficiently flexible to effect a seal between it and the patient's ear canal.

Further features of the invention will be described or will become apparent in the course of the following detailed description.

### BRIEF DESCRIPTION OF THE DRAWING

In order that the invention may be more clearly understood, the preferred embodiment thereof will now be described in detail by way of example, with reference to the accompanying drawings, in which:

Figure 1 is a cross-section of the preferred embodiment in its slim state, ready for insertion in the ear canal;

Figure 2 is a cross-section of the same embodiment in its expanded state;

Figure 3 is a cross-section of an alternative embodiment; and

Figure 4 is a drawing showing a mechanism used to ordinarily hold the device in its unexpanded position.

### DESCRIPTION OF THE PREFERRED EMBODIMENT

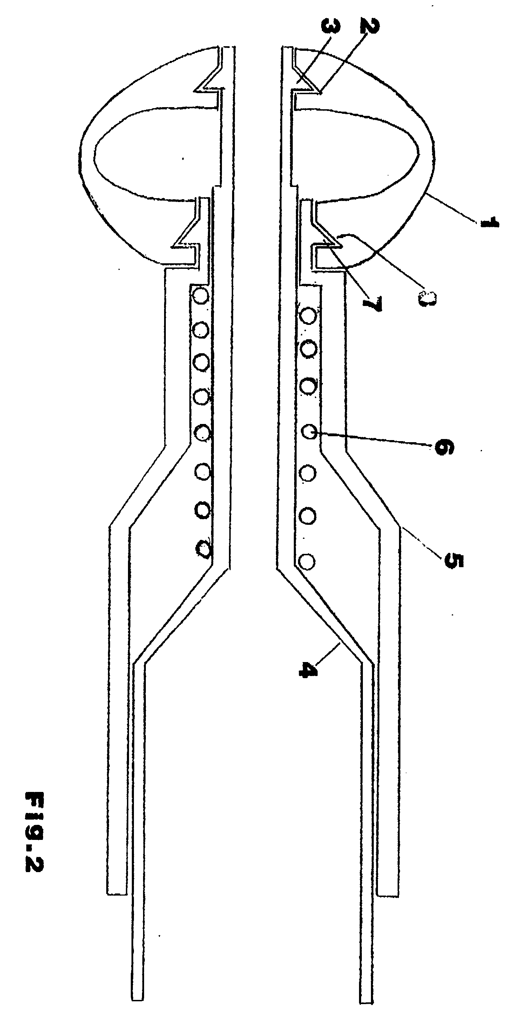

Referring now to the drawings, the device has a cuff 1 made of rubber or other soft plastic material. The cuff is formed in such a way that it will give maximum expansion of the diameter when as shown in Figure 2.

The softness is chosen so that it will give enough pressure in the ear canal to give an airtight seal but soft enough not to feel too uncomfortable.

The cuff has an annular channel 2 which fits over a flange 3 near the end of the probe 4 to ensure that it is firmly secured to the probe to avoid having the cuff remain in the ear canal after the probe has been retracted. The cuff should be made of material that does not create allergy problems.

The device has a probe element 4 for impedance measurements. It can have the receiver for ipsilateral stimulation built in, as well as necessary facilities for connecting the sound source, the microphone and tubing for air pressure.

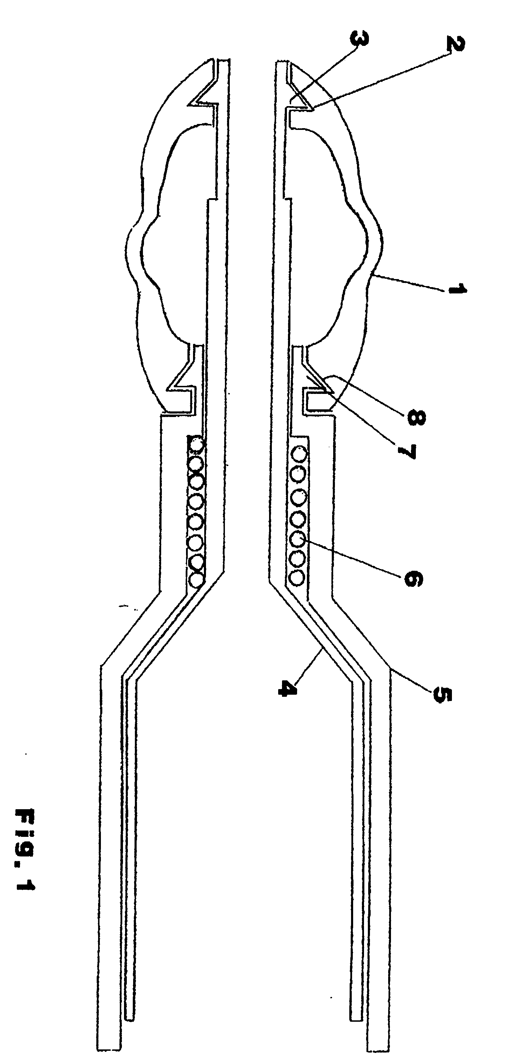

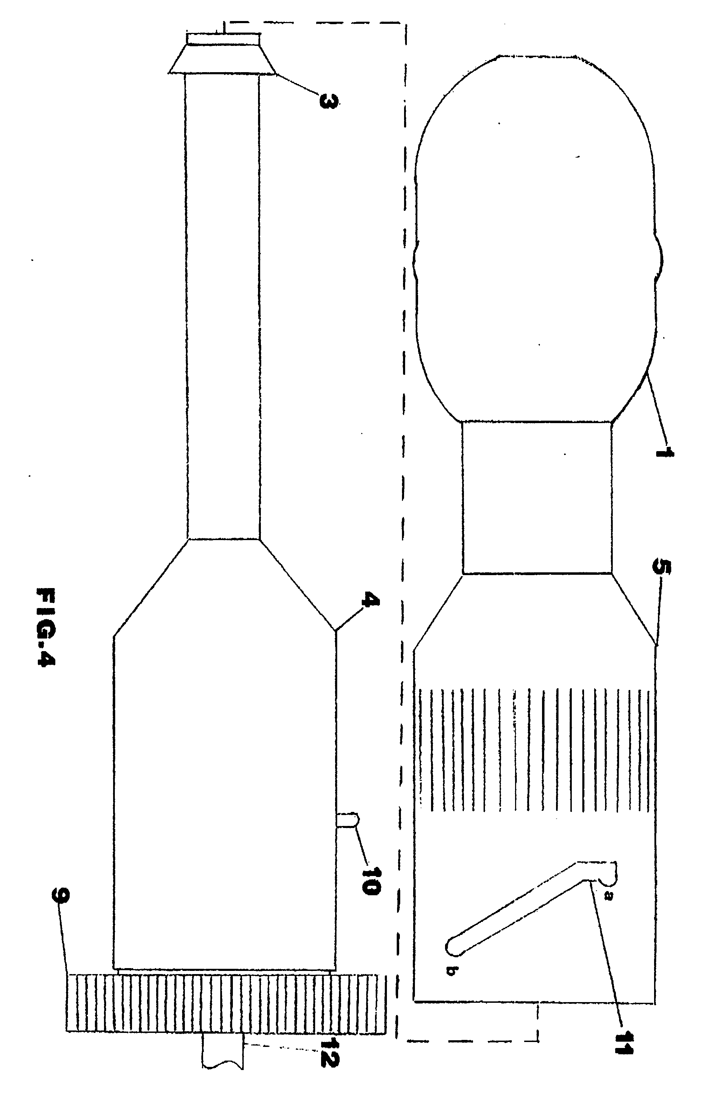

The actuator 5 is used to change the pressure on the cuff in order for it to expand. The actuator can be spring-loaded as shown in Figures 1 and 2, by virtue of a spring 6. The actuator engages the cuff with a flange 7 which fits into a channel 8 in the cuff. A mechanism as shown in Figure 4 acts to ordinarily hold the device in its neutral or unexpanded position as shown in Figure 1. This position is used during insertion into the ear canal. Once inserted, the actuator is released and the spring applies pressure to the cuff, expanding the cuff to the diameter of the ear canal it is inserted into. In the mechanism shown in Figure 4, the collar 9 is normally a nylon or other plastic part of the probe containing a receiver (speaker) and tubing for connection the microphone and air pump. These connections come to the probe via the cable 12.

To the probe is added a guide 10 which fits into the slot 11. When the actuator 5 is fitted to the probe 4 as shown in Figure 1, the guide will be at position A in slot 11. When the actuator is turned in the direction of the arrow, the spring 6 will push the actuator forward and make the cuff expand.

Instead of a spring-loaded actuator, the actuator can have facilities to make it move by turning. The actuator and probe could have coarse threads or other facilities that will make the actuator to move forward on the probe.

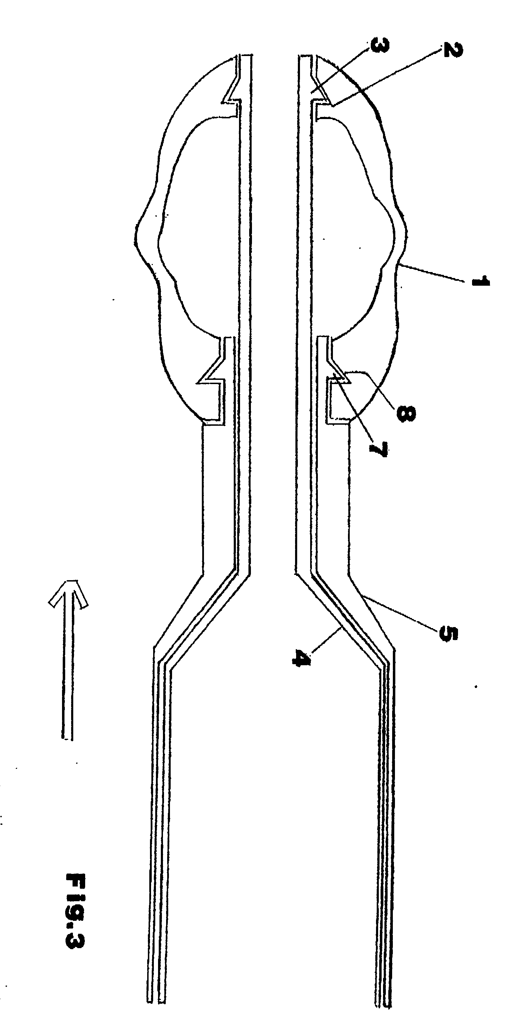

Another alternative, especially useful for screening tests, is to have the actuator freely moveable on the probe, with no spring, as shown in Figure 3. The probe is inserted into the ear canal, with one only holding on to the actuator and pushing towards the ear canal. By doing this the cuff will bulge and make a seal.

This invention address the problems of the prior art described above. The cuff is made in such a way that the distal end is fixed at the tip of the probe. The other end is fixed to an actuator that can be spring-loaded or have coarse threads so it can be turned and squeeze the cuff or be activated in any other way. The cuff can be made in different sizes and it is believed that three sizes will cover 98% of ears from the age of 3 to 80 years. When the cuff is squeezed it will bulge and fill the ear canal and form a seal whether the ear canal is circular or elliptical. The probe with this form of cuff is very easy to insert into the ear canal as the diameter of the cuff can be less than the diameter of the ear canal. It is only when the activator is activated that the cuff bulges and fills the ear canal. This also secures the probe so that it does not fall out of the ear canal but sits firmly during the test.

It will be appreciated that the above description relates to the preferred embodiment by way of example only. Many variations on the invention will be obvious to those knowledgeable in the field, and such obvious variations are within the scope of the invention as described and claimed, whether or not expressly described.

## Claims

1. A probe for use in acoustic impedance measurement, comprising:
- a probe element having a distal end for insertion in a patient's ear canal;
- an actuator coaxial with and outside said probe element, said actuator having a distal end not extending as far as the distal end of the probe element; and
- a flexible cuff outside and extending between said distal ends of said probe element and said actuator;
said actuator being moveable axially with respect to said probe element between a first position where said distal ends are axially separated such that said cuff is in an unexpanded minimum diameter state and a second position where said distal ends are closer together such that said cuff is forced to an expanded larger diameter state, said cuff being sufficiently flexible to effect a seal between it and the patient's ear canal.

Fig. 1

Fig. 2

Fig.3

FIG.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | US-A-4 029 083 (C.S. BAYLOR) * column 4, line 46 - column 5, line 46; column 5, line 67 - column 6, line 24; figures 1-4 * | 1 | A 61 B 5/12 |
| A | US-A-4 289 143 (F. CANAVESIO et al.) * column 2, line 56 - column 4, line 26; figure 1 * | 1 | |
| A | US-A-4 327 905 (J.E. KELLER et al.) * column 3, line 41 - column 4, line 26; figure 1 * | 1 | |

|  | | | TECHNICAL FIELDS SEARCHED (Int. Cl.5) |
|---|---|---|---|
| | | | A 61 B 5/00 A 61 F 11/00 G 01 H 15/00 |

The present search report has been drawn up for all claims

| Place of search | Date of completion of the search | Examiner |
|---|---|---|
| BERLIN | 13-08-1990 | WEIHS J.A. |

EPO FORM 1503 03.82 (P0401)